# EUROPEAN PATENT APPLICATION

(11) **EP 1 847 317 A1**
(43) Date of publication of application: **24.10.2007**
(21) Application number: 07106545.2
(22) Date of filing: 19.04.2007
(51) Int. Cl.: B01J 19/00, C12Q 1/68, G01N 33/543

(54) **Method for stabilizing functional groups on a surface of a polymer used as solid support for making microarrays**

(30) Priority: 19.04.2006 EP 06112775
(71) Applicant: Eppendorf Array Technologies SA, 5000 Namur (BE)
(72) Inventor: Remacle, José, B-5020 Malonne (BE); Hamels, Sandrine, B-1474 Ways (BE); Koehn, Heinz, 22339 Hamburg (DE)
(74) Representative: pronovem

(57) **Abstract**

The present invention concerns a new and simplified method for obtaining a polymer surface activated with a high density of aldehyde functions which are stable with time and ready to react covalently with amino groups present on a great number of different molecules used as capture probes according to a microarray.

## Description

### Field of the invention

The present invention is related to a stabilization method of functionalized chemical surfaces used for the construction of microarray upon which capture molecules (nucleic acids, proteins) are covalently fixed.

### Background of the invention

Microarrays are powerful tools for simultaneous detection of many different target molecules present in a sample, preferably biomolecules like nucleotide sequences, ligands, antibodies, etc. For DNA biochips, binding properties of molecules present upon an array depend mainly on the number, the sequence and the length of capture nucleotide sequences and the way they are addressed onto a solid support. DNA biochip technology uses microscopic arrays of DNA molecules immobilised on solid supports. Biochips microarrays applications are numerous and used for biomedical analysis such as gene expression analysis, polymorphism or mutation detection, molecular diagnostic, DNA sequencing and gene discovery (Ramsay et al., Nature Biotechnology 16, p. 40 (1998)).

Such DNA microarrays are prepared by various methodologies. DNA can be synthesised *in situ* on glass surface by using combinatorial chemistry (Pease et al., Proc. Natl. Acad. Sci. USA 91, p. 5022 (1994)). This methodology produces DNA microarrays consisting of groups of oligonucleotides ranging in size from 10-25 bases whereas DNA microarrays prepared by micro-deposition with a robot can be of any length going from small oligonucleotides to 0.5-2 kb nucleotide sequences obtained for example after amplification by the polymerase chain reaction (PCR) (Zammatteo et al., Anal. Biochem. 253, p. 180 (1997)). Mechanical microspotting uses passive (pins) or active (ink jet nozzles) devices to deliver small quantities of DNA onto known regions.

Glass is a popular substrate for DNA biochip, primarily due to its low fluorescence, transparency, low cost and resistance to high temperature and many chemical reagents (Cheung et al., Nature Genetics supplement 21, p. 15 (1999)) .

Zammatteo et al. (Analytical Biochemistry 280, p. 143 (2000)) compared several coupling strategies currently used to covalently graft DNA onto glass surface. They tested the carbodiimide mediated coupling of aminated, carboxylated and phosphorylated DNA on carboxylic acid or amine modified glass supports. These methods were compared with the binding of aminated DNA to aldehyde activated glass. They concluded that the fixation of aminated DNA to aldehyde modified surface gives the best coupling procedure to build DNA microarray in term of coupling yield, rate of reaction in the absence of coupling agent. WO02/18288 describes a method for obtaining a surface of glass derivatized with aldehydes for building microarrays.

Besides glass, polymers are becoming increasingly used for microarray and for the miniaturisation of the biological assays due to the development of the microfluidic technology and the "lab on a chip" concept. In order to perform the assays, biological or ligand molecules have to be fixed on the surface of the polymer and the requirement of a simple method of polymer activation would be valuable.

Polymer activation is easily and quickly obtained by physical and/or chemical treatment such as radio frequency (RF) plasma or plasma treatment. The problem of such activation process is that a rearrangement of functional groups occurs on the surface of the polymer. The reason is due to the fact that polymers lockboxes or branches are flexible and may rearrange, some groups being incorporated inside the polymers and other outside.

However, it is difficult to predict the importance and rate of this rearrangement, because it meanly relies on the nature of the polymer, of the breaks, of the length of the lockboxes, of the functional groups, of the interactions between the groups on the surface and in the inner part of the polymer.

Because of this rearrangement, the density of functional groups available on the surface of the polymer support is difficult to estimate and to control since their number changes with time. Also functional groups which are the most reactive and useful for fixation of molecules usually show bad stability with time. For instance, by storage in air, amino groups can be lost due to surface reorganization and to oxidation of primary amines to amides (Gegenbach et al. 1994, Adhesion Sci. Technol. 8, 305).

Therefore, it is difficult to make reproducible fixation of biological reactor, such as capture probes on microarray where high number of functional groups has to be available on the surface of the polymer in a constant density, in a reproducible and uniform distributed manner in order to be useful as a method for industrial production of microarray.

Time is a parameter difficult to control in the production of microarray since making functionality and deposition should be controlled in a strict time scale which is not easy for large production. Functionalized surface are produced in bulk and are stored until their use for industrial microarray production.

There is a requirement for providing a method capable of introducing stable functional groups on the surface of polymer in order to use the functionalized polymer for making microarray.

### State of the art

The US Patent Application US 2004/068882 and US 2005/059083 describe a method for immobilizing ligand binding polypeptides preferably antibodies to a modified solid support surface by a plasma treatment to prevent non specific cell and protein adsorption. In the described method, the surface of the solid support is first modified by a plasma treatment to generate the formation of amino groups upon which hyaluronic acid or alginic acid can be bound to the surface. When this acid molecule is bound to the solid support surface, free hydroxyl groups of acid molecule are oxidized to aldehydes, for example with addition of periodate. Thereafter, the polypeptide can react with an aldehyde group through its free primary amino group. This method can be done in the form of microarray patterm of dots.

This method requires firstly a modification of the acid molecule in order to create free hydroxyl group upon the bounded acid molecule. This acid molecule being bound upon the surface of the solid support by the addition of a coupling agent for obtaining a binding between the generated amino groups of the solid support and this acid molecule.

### Aims of the invention

The present invention aims to provide a new and simplified process (method) for obtaining a polymer surface activated with a high density of aldehyde functions which are stable with time and ready to react covalently with amino groups present on a great number of different molecules used as capture probes according to a microarray.

A preferred aim of the present invention is to provide by said method, microarrays at the bottom of the wells of microtiter plates as an alternative to standard glass slides and to allow automatization and high throughput screening of target molecules upon these microarrays allowing their identification and/or quantification and/or recovery.

### Summary of the invention

The present invention is related to a method for obtaining microarrays upon a polymeric solid support. This method comprising the following (preferably successive) steps:
- activating a surface of the polymeric solid support by a plasma treatment to allow the formation of amino groups (preferably comprising primary amino groups);
- providing a macromolecule bearing multiple aldehyde groups (preferably a macromolecule which has a high molecular weight, preferably comprised between 10000 and 1000000 more preferably between 50000 and 100000, more preferably about 60000 daltons, this macro molecule being preferably an oxidised polysaccharide, but does not comprise any acid group, more preferably, this macromolecule being a dextran or a agarose);
- covalently binding the said macromolecule on the surface of the said solid support, said macromolecule being immobilized by at least one shift base or imine group, and,
- covalently fixing upon the free aldehyde groups of the immobilized macromolecule (these free aldehyde groups being the ones of the immobilized macromolecule which have not been reacted with the amino groups present upon the activated solid support surface)capture molecules carrying one or more amino groups, by a deposition of a solution containing the capture molecules on the solid support surface, wherein the covalent fixation results in an array comprising a density of at least 4 discrete regions per cm² of solid support surface, each of said discrete surface regions being fixed with a species of capture molecules.

The present invention is also related to a method for obtaining microarrays on a polymeric solid support surface comprising the steps:
- activating a surface of the polymeric solid support by a plasma treatment in order to allow the formation of amino groups comprising primary amino groups;
- providing a macromolecule bearing multiple aldehyde groups,
- covalently binding the macromolecule on the surface of the solid support, wherein said macromolecule is immobilized by at least one shift base or imine group,
- covalently fixing upon the free aldehyde groups of the immobilized macromolecule, capture molecules carrying one or more amino groups by a deposition of a solution containing the capture molecules in one discrete region of the solid support,
- repeating the solution deposition until a microarray is constructed with different capture molecules being present in different discrete regions and/or having the same capture molecule in replica discrete regions wherein the microarray comprises a density of at least 4 discrete regions per cm² of solid support surface, and wherein the amount of capture molecules fixed in one discrete region varies by less than 25%, and preferably less than 10% as compared to the amount fixed in a replica discrete region.

In the method according to the invention the amount of capture molecules fixed in one discrete region varies by less than 25% and preferably less than 10% as compared to the amount of fixed in a replicate discrete region. This condition could be obtained in the method according to the invention by obtaining a covalent fixing upon the free aldehyde groups with a first selected capture molecule carrying one or more amino group(s) by a deposition of the solution containing the said selected capture molecule on the solid support surface and by submitting the same solid support surface in another discrete region to a similar covalent fixing step of a replica, the replica comprising the same solution containing the same capture molecule, but being deposited in another region on the solid support surface with the same conditions (the same volume and the same physical parameters) (i.e. by using the same pin the same contact between the pin and the solid support surface, so that only the location (the discrete region) differs between the fixing of a capture molecule and its replica).

The locations of the discrete regions (or spots) have a diameter comprised preferably between about 10 and about 500 µm and are separate by distances of similar order of magnitude, so that the array of the solid support surface comprises a density of between about 10 and about 250000 discrete regions or spots per 1 cm², and so preferably comprised a density higher than 20, or so and even higher than 100 or 250 and even higher than 1000 or 5000 spots per cm².
Also the surface of the discrete regions fixed by capture molecule is less than 2 mm² and even less than 0.1 mm2 and even less than 0.01 mm².
The array is constructed by with different capture molecules being present in different discrete region and/or having the same capture molecule in more than one discrete region (replica of discrete regions).

The polymeric solid support according to the invention is a solid support suitable for microarrays, especially for "biochips" or "chemochips", comprising at specific locations fixed capture molecules such as capture nucleotide sequences, capture antigens, capture antibodies or hypervariable portion(s) thereof, capture ligands, capture receptors, capture aptamers, (designed to be) specific for complementary molecules such as target nucleotide sequences, target antibodies, target antigenic structures, target receptors or target ligands to be detected, quantified and/or recovered from a sample.

The microarrays are also suitable for the preparation of chemochips based upon the same principle for the detection, the quantification and/or the recovering of specific chemical molecules, such as molecules obtained by combinatorial chemistry.

The method is provided so that the fixation of the capture molecules after 1 and better 3 months does not decrease more than 25 % when the support is kept at 4°C. The fixation of the capture molecule after 1 and better 3 months does not decrease more than 25% when the support is kept at 40°C.

The capture molecules are designed for a detection, an identification and/or a quantification of complementary target biological or chemical molecules of interest being present in a biological sample.

The method of the invention can obtain high density of capture molecules fixed on the support with at least 20 fmoles, preferably 100 fmoles and better 200 fmoles of polynucleotide capture probes fixed per cm².

In the method of the invention, the fixation of the capture molecules on the support does not require any additional chemical activation molecule present in the deposition solution. Preferably, the capture molecule is present in an aqueous (deposit) solution.

In the method of the invention, the fixing of the capture molecule(s) on the support is performed within about 10 and better about 5 and even better about 2 min. and with the obtained microarray the fluorescence detection signal of the solid support surface (background) is decreased by a factor of at least 4, after the binding of the macromolecule compared to the plasma activated polymer.

In the method of the invention, the activating step (the formation of amino groups into the polymeric solid support surface) is obtained by a plasma treatment. This physical treatment presents several advantages over chemical treatment. Chemical treatment usually involves the use of organic solvents which alter the polymer, especially its transparency property. Plasma treatment is an environmental friendly technique, because of the low energy consumption, the fact it is a dry technique (no additional drying step), no waste disposal problem and disposal cost. Contrary to a chemical treatment, a plasma treatment is an operator friendly technique (no chemical products used). A plasma treatment is a qualitative and full controllable process (all parameters are controlled by the unit and quality control possible by print-out and data-logging). It is also a very efficient treatment (higher degree of activation, longer shelf-life than alternative methods as corona and flaming). There is no substrate damage or bulk properties changes and there is no limit to substrate geometries: small or large, simple or complex (slide, microtiter plate, etc.).

Preferably, in the method according to the invention, the solid support comprises or is made of a (preferably transparent) polymer selected from de group consisting of PS (polystyrene), PC (polycarbonate), PMMA (Polymethyl methacrylate), PE (polyethylene), COC (Cycloolefin copolymer) or COP (cyclic olefin polymer). A preferred COP product is Zeonex^{™} because of its excellent optical properties, chemical resistance, thermal stability, low fluorescence. When the binding reaction between the target molecule and capture molecule requires high temperature incubation, the solid support is preferably thermo-resistant at 100°C like PC or COP.

Preferably, the solid support comprises multiple microarrays surfaces disposed according to a multiple well microtitre plate format, said format being preferably selected from the group consisting of 6-wells, 12 wells, 24-wells, 48-wells or 96-wells formats.

Preferably, the activation step of the surface of the polymeric solid support allowing the formation of amine functions is obtained plasma treatment using N-containing gases (like ammonia or nitrogen or a composition thereof) (Lub et al. 1989, Polymer, 30, 40-44; Nakayama et al. 1988, J. Polymer Science, 26, 559-572). The activation step of polymeric solid supports can be done by the person skilled in the art according to a method well-known described in the scientific literature and illustrated in the example 1.

The introduced amine functions are identified upon the solid support by X-ray photoelectron spectroscopy (XPS) analysis.

Preferably, the stabilization step of the surface of the polymeric solid support allowing the formation of aldehyde functions is obtained by addition of a macromolecule (bearing multiple aldehyde groups) to the polymeric surface derivatized with amino groups.

Preferably, the macromolecule bearing aldehyde groups has a molecular weight comprised between about 10000 (daltons) and about 600000 (daltons), preferably between about 50000 (daltons) and about 100000 (daltons).

The inventors found that the same macromolecule could advantageously provide aldehyde groups that allow a stabilization of the amino groups and for a covalent binding of capture probes designed for a microarray assay, making the production of these microarray assays easy to perform.

The invention provides a very reproducible method which is well adapted for product manufacturing on plastic surface and on a large scale. Advantageously, the spotting of capture molecules on the aldehyde group(s) surface can be performed a certain time after obtaining the aldehyde group(s) surface without loosing the binding capacity and reproducibility of the capture molecules spotting, preferably 1 week, even preferably 15 days, even better 1 month, even better 3 months and even more than 6 months after the treatment. In this experiment, the hybridization capacity of capture molecules spotted at 300 nM on the Zeonex™/polyaldehyde prepared according to the present invention was compared to the same molecule spotted in the same conditions onto a reference material being a glass slide functionalized with aldehydes (diaglass) under the process disclosed in WO02/18288.

Capture molecules fixed on the two different materials of the solid support surface have similar hybridization efficiencies. The same signal of intensity compared to the diaglass indicates that the present invention provides capture molecules being fixed at a high density since the signal intensity of the hybridization of a target is dependant on the density of the capture molecules present on the solid support surface. In WO02/18288, the inventors have shown that a high density of aldehyde functions was present on the glass slide and allowed the grafting of as much as 230 fmoles of capture molecules per cm² when spotted at a concentration of 300 nM. Since the hybridization signal is dependant on the density of the capture molecules on the spots, the inventors have estimated that the density of capture molecules immobilized on the functionalized Zeonex^{™} is in the same order of magnitude as observed on the diaglass slides which means that the method of the present invention can provide an activated surface on the polymer solid support that will bind polynucleotide capture molecules at a density of about (around) 230 fmoles of capture molecules per cm2 when performed in the appropriate conditions of molecules spotting.

The detection of target molecules on spots being present in different locations on the array have showed very low variability with CV lower than 25% and even lower than 10% for replicated spots indicating the uniformity of the capture molecules distribution and of the chemistry on the overall surface treated as provided by the present invention. This homogeneity characteristic was not known nor predicted by documents of the prior art and makes the invention advantageous as compared to the other proposed activated surfaces.

The used macromolecule in the present method is preferably an oxidized polysaccharide (preferably dextran or agarose). Oxidation of dextran is well documented in the literature (Azzam et al. 2002, J. Med. Chem, 45, 1817-24; Azzam et al. 2002, Macromolecules, 35, 9947-53) and is preferably performed using the periodate oxidation.

According to the invention, the target molecules may be present in a sample (biological sample), such as a clinical sample extracted from blood, urine, vessels, saliva, pus, serum, tissue, fermentation solutions or culture media. Said target compounds are preferably isolated, cleaved, purified and/or amplified (if necessary) by known methods by the person skilled in the art before their detection and/or quantification upon the microarrays according to the invention.

Therefore, the capture molecules present upon the microarrays are specific for these complementary target molecules and are preferably parts of coupling pairs, such as complementary strands of nucleotide sequences, antibodies (or active hypervariable portions of an antibody/antigenic structure or haptens) receptors/ligands, biotin/streptavidin molecules, possibly coupled with other chemical or biochemical molecules or any double pairs binding system suitable for the identification, characterisation, screening and recovery of biological or chemical libraries of molecules, for biomedical analysis such as gene expression analysis, polymorphism or mutation detection, molecular diagnostic, DNA sequencing and gene characterisation.

The present invention is also relates to a polymeric solid support used for making microarray, having fixed on its surface macromolecules bearing (free) aldehyde functional groups, these macromolecules being immobilized on the surface of the polymeric support by at least one shift base or imine groups. Preferably, the macromolecule has a molecular weight comprised between about 10000 (daltons) and about 600000 (daltons), preferably between about 50000 (daltons) and about 100000 (daltons).

The invention also relates to a microarray obtained by method described herein comprising polymeric solid support surface on which capture molecules carrying one ore more amino groups are covalently bound on this macromolecule being previously bound to the surface of the polymeric support by at least one shift base or one or more imine group(s), this covalent fixation resulting in an array comprising a density of at least 4 discrete regions/cm² of this solid support surface, each of the discrete surface regions being fixed with a species of capture molecules. On the solid support surface, the capture molecules are designed for the detection, the identification, the quantification and/or the recovery of complementary target biological or chemical molecules of interest. In a preferred embodiment, the density of the capture molecule fixed on the support is more than 20 fmoles and better 200 fmoles, or even 1000 fmoles of capture probes per cm².

The present invention will be described in details in the following non-limiting examples in reference to the enclosed drawings.

### Short description of the drawings

Fig. 1 gives a schematic presentation of the process for production of a polyaldehyde polystyrene surface. The first part of the process is a NH₃ plasma treatment which introduces amine functions into the polystyrene surface. In a second part of the process, oxidized dextran is covalently fixed on the aminated support, providing free aldehyde functions.

Fig. 2 gives the fixation capacity of a concentration curve of DNA on polyaldehyde polystyrene surface obtained by the process of figure 1. The surface provided as negative control corresponds to non-plasma treated PS incubated with dextran 1%.

Fig. 3 gives the hybridization capacity of DNA nucleotide sequences immobilized at 300 nM on polyaldehyde polystyrene surface obtained by the process of figure 1. The negative control is the same as in figure 2.

Fig. 4 gives the binding capacity of antibodies immobilized on polyaldehyde polystyrene surface obtained by the process of figure 1. The negative control is the same as in figure.

Fig. 5 shows general XPS spectrum of non treated COP Zeonex^{™} (reference) compared to the polymer treated by N₂ plasma.

Fig. 6 gives the stability of polyaldehyde COP Zeonex^{™} surface with time. Capture molecules have been spotted and hybridized with target DNA in the same conditions after different times of storage of the Zeonex^{™} polyaldehyde at 4°C and 40°C. A surface treated by NH₃ plasma only is provided as negative control. A glass slide functionalized with adlehydes (diaglass) is provided as positive control.

### Definitions

The term "polymer" or "polymeric solid support" is a generic term used to describe a substantially long molecule. This long molecule consists of structural units and repeating units strung together through chemical bonds. The process of converting these units to a polymer is called polymerization. These units consist of monomers, which are typically small molecules of low molecular weight. The term "polymer" as used in the present invention excludes biopolymers such as proteins and nucleic acids which require the help of catalysts for their formation.

The term "macromolecule" means a giant molecule that contains at least several hundred atoms, a size much larger than simple molecules, like water or glucose. Many molecules that are an integral part of living things, such as DNA and proteins, are macromolecules. Knowing the three-dimensional structure and sequence of the groups of atoms in macromolecules is crucial in understanding how they react biochemically.

The term "Functional group" means an atom or group of atoms, such as a carboxyl group, that replaces hydrogen in an organic compound and that defines the structure of a family of compounds and determines the properties of the family. Organic compounds are frequently classified according to the functional group or groups they contain. For example, methanol, ethanol, and isopropanol are all classified as alcohols since each contains a functional hydroxyl group. Functional groups includes without being limited to: Hydroxyl (R-OH), Methyl (R-CH₃), Alkene (R-CH=CH-R'), Alkyne (R-C≡C-R'), Amide (R-C(=O)N(-H)-R'), Amines (primary amine, R-NH₂; secondary amine, R-N(-H)-R'; tertiary amine, R-N(-R')-R)), Azo (R-N=N-R'), Nitrile (R-C≡N), Pyridyl (R-C₅H₄N), Carboxyl (R-C(=O)OH), Aldehyde (R-C(=O)H), ketone (R-C(=O)-R'), imines (primary imine, R-C(=NH)-R'; secondary imine, R-C(-H)=N-R'), Ether (R-O-R'), Ester (R-C(=O)O-R'), Halogen (F,Cl,Br,etc.), Isocyanate (R-N=C=O), Isothiocyanate (R-N=C=S), Phenyl (R-C₆H₅), Benzyl (R-CH₂-C₆H₅), Phosphodiester (R-OP(=O)₂O-R'), sulfhydryl group (R-SH), Thioether (R-S-R'). The non-hydrogen atoms of functional groups are always associated with each other and with the rest of the molecule by covalent bonds.
The term "array" or "Micro-array" means a solid support surface on which multiple capture molecules are fixed in order to be able to bind to the given specific target molecule. The micro-array is preferentially composed of fixed capture molecules present at specifically localized areas on the surface or within the support or on the substrate covering the support. A specifically localized area is the area of the surface which contains fixed capture molecules specific for a determined target molecule. The specific localized area is either known by the method of building the micro-array or is defined during or after the detection. A spot is the area where specific target molecules are bound on their capture molecules and seen by the detector. In one particular application of this invention, micro-arrays of capture molecules are also provided on different or separate supports as long as the different supports contain specific fixed capture molecules and may be distinguished from each other in order to be able to quantify the specific target molecules. This can be achieved by using a mixture of beads having particular features and being able to be recognized from each other in order to quantify the bound molecules. One bead or a population of beads is then considered as a spot having a fixed capture molecule specific of one target molecule. Also the multiwells bearing in each well a capture probes are then considered as an array.
Microarrays are preferentially obtained by deposition of the capture molecules on the substrate is done by physical means such as pin or "pin and ring" touching the surface, or by release of a micro-droplet of solution by methods such as piezo or nanodispenser. Alternatively, *in situ* synthesis of capture molecules on the substrate has also been described as by 5,744,305 and 6,346,413.

As used herein, "capture molecule" refers to a molecule, or complex or combination thereof, that is capable of specifically binding to one target molecule, or to a family of target molecules, or to one or more member (s) of a plurality of target molecules, or portion(s) thereof. The capture molecules are preferably nucleic acids being oligonucleotides or polynucelotides which are either synthesized chemically in situ on the surface of the support or laid down thereon. Nucleic acid binding is achieved via base pairing between two polynucleotides, one being the immobilized capture molecule and the other one the target to be detected.

The term "Replica" of discrete region means the same solution containing the capture molecule is deposit in another region of the support in the same conditions preferably the same volume and the same physical parameters. In the case of the spotting by physical contact such as with a pin, the replica means the same solution and the same pin is used with the same physical parameters for the contact between the pin and the support so that only the location of the deposition differs between two replicas of discrete regions. With contactless deposition the same solution and physical parameters for providing the liquid droplet deposition on the support is used for making replicas.

A macromolecule bearing multiple aldehyde groups means a macromolecule of a high size which comprises high number of aldehyde groups(preferably more than 10, more than 100, more than 1000 or more than 10000 aldehyde groups) to allow a binding between some of these aldehyde groups with amino groups present upon the activated polymeric solid support surface, and to allow a binding of the other free aldehyde groups of this immobilized macromolecule to amino group(s) of capture molecules.

### Detailed description of the invention

One constraint of a biochip is that detection of the biological molecules upon said biochip is performed either by colorimetric or fluorescent methods. Plastic polymers physical properties like transparency or fluorescence are easily altered by using organic solvents. This drawback exists also with polycarbonate plastic used in a compact disc support (CDs), which is easily altered by organic solvents.

The invention is easily applicable to most polymeric material, either if they contain amino groups or by incorporating these amino groups at a surface of a support by physical crosslinking of amino group at the surface of the support.

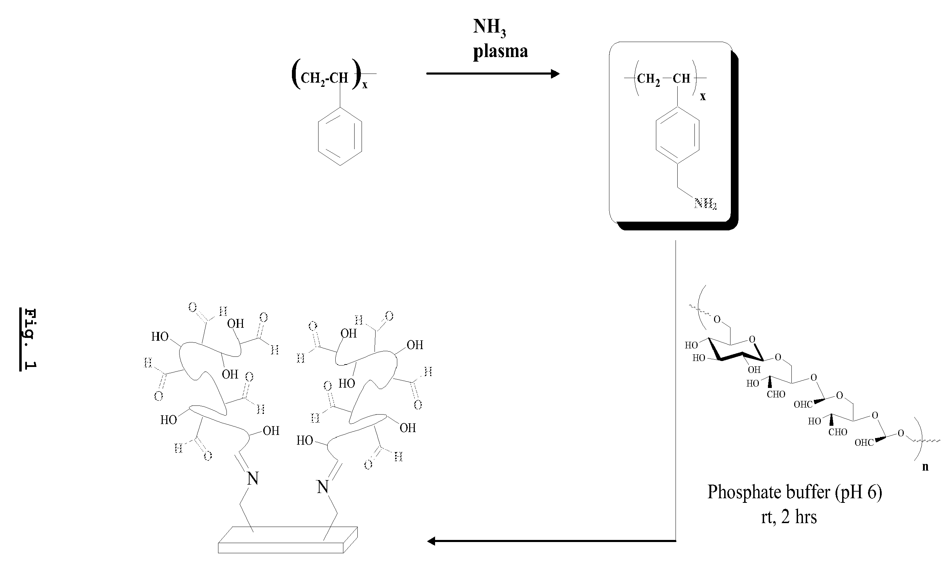

In one preferred embodiment of the invention, poly-cycloolefin is used as support for biochip microarrays construction by first attachment of amino groups by an ammonia plasma treatment and then a stabilization of these amino groups into generated aldehyde groups (cf. Figure 1). Preferably, the use of (organic) macromolecules or polysaccarides of different sizes are used to adapt the number of generated (free) aldehyde groups and the availability for a fixing of capture probes of different sizes.

In another embodiment of the invention, biological capture molecules are fixed to the surface with the generated aldehyde groups and correspond to the first member of a binding pair (capture molecule). The second member is the molecule to be detected or identified or quantified (target molecule) in biological or chemical samples.

Preferably, the first member is an antigen (hapten) or antibody, a ligand or a receptor, a biotin or a streptavidin but also peptides, proteins or nucleic acids (single or double strand DNA or RNA fragments) which are recognised by a complementary or other binding molecules. For example double stranded DNA specific sequences attached to a support can be used to detect DNA binding proteins. One specific application is the detection of transcriptional factors.

The invention is particularly well suited for construction of large number fixed capture molecules on the same surface and its automation. Thus libraries of chemicals, peptides, ligands, antigens capture molecules are easily constructed on such support given the facility of capture molecules fixing by a robot. The obtained solid supports are then easily used for screening libraries of target molecules either biologically (like clones, plasmids bank or phage display molecules) or chemically constructed. Chemical libraries are now easily constructed due to the progress in the combinatorial or parallel synthesis of target molecules.

### Examples

### Example 1: preparation of polystyrene polyaldehyde by physical process

### 1. Preparation of aminated Polystyrene

The primary amines functions are introduced into the polystyrene by ammonia plasma treatment.

Surface modifications were performed by conventional low-pressure rf plasma discharges in NH₃ plasma. Polystyrene 96-well plates were positioned on trays and trays were placed into the chamber. The electrodes have the same size than the trays, so that the samples are covered and treated homogeneously. Distance between electrode and sample is 8 cm. After introduction of the samples in the reactor chamber (W 305 mm, H 300 mm, L 370 mm) and pumping down to 8 x 10⁻² mbar (vacuum pump: Leybold, Type D16B), the gaz flow was started and the plasma discharge was performed (working pressure 0.3 mbar, 40 kHz Generator with 30 % power; discharge time 5 min).

### 2. Preparation of dextran polyaldehyde

Dissolve 2.5 g of dextran (Molecular Mass 70000 ; Aldrich n° D1537) in 50 ml distilled water, then add 3.594 g of potassium periodate (15.6 mmol ; Aldrich n° 322423). Shake vigorously for 14 h at room temperature in the dark and dialyze for 3 days at 4°C (cut-off of 10000; 3 times 1 litre of distilled water). The solution is centrifuged and lyophilized. 2.15 g of dextran polyaldehyde are obtained (yield: 86%), which can be stored at room temperature until use.

0.125 g of dextran polyaldéhyde are dissolved in 12.5 ml of phosphate buffer 0.1 M pH 6. The mixture is heated a few minutes at 60°C under vigorous stirring until complete dissolution (final solution 1 %). Let the solution cooling until room temperature before use.

### 3. Preparation of Polystyrene polyaldehyde

Add 70 µl of dextran polyaldehyde solution obtained at step 2 in each well of a 96-well polystyrene plate carrying amino groups. Cover the plate with a lid and incubate for 2 h at room temperature. Wash 3 times with distilled water. Store the 96-well plate under vacuum until use.

### Example 2: Binding capacity of polystyrene polyaldehyde compared to non treated polystyrene

### 1. Capture probe immobilization

Chlamydia DNA sequences are used as capture nucleotide sequence. Capture nucleotide sequences are synthesized by PCR using one primer carrying primary amine function at 5' end. Biotinylation is carried out by the incorporation of biotin-dCTP during the PCR amplification. Aminated capture nucleotide sequences are diluted to a concentration of 300, 100, 30, 1 and 0.3 in SSC2X, sarcosyl 0.0025% and are dispensed at the bottom of wells of polyaldehyde microtiter plates (MTP) with an arrayer. Polyaldehyde MTP are obtained either as described in example 1. MTP used as negative control corresponds to non-plasma treated (non-aminated) PS incubated with dextran polyaldehyde. The pins used for spotting into the wells of the MTP are split pins (n° 1545 Genetix Limited). Each array is composed of 18 spots (9 X 2). Each aminated probe concentration (including the negative control) is fixed in triplicate. After the spotting, the wells of the MTP are washed once for 1 min with 0.2% SDS, twice with distilled water. The wells are then incubated for 5 min with NaBH₄ solution (2.5 mg/ml of PBS 75%/ Ethanol 25%), washed twice with distilled water and dried. MTP are stored under vacuum at 4°C.

### 2. Colorimetric detection

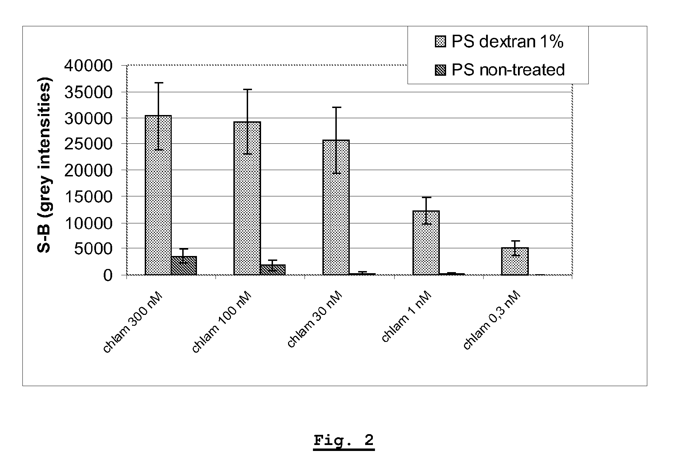

Place the MTP on a thermomixer (Eppendorf) at a speed of 300 rpm and incubate 45 min at room temperature in conjugate Anti biotin-gold diluted 500x in blocking buffer (Eppendorf), 100 µl/well. After incubation, wells are washed 4 times 1 min with washing buffer (Eppendorf), then once with rinsing buffer, incubated 5 min at room temperature with Silverquant A and B solutions (Eppendorf), then rinsed twice with water, dried and analysed using MTP scanner (Eppendorf, Hamburg, Germany). Each slide were then quantified by the silverquant Analysis software. Results are presented in figure 2. The MTP used as negative control (non-aminated PS incubated with dextran polyaldehyde) gives a much lower binding capacity.

### Example 3: Hybridization capacity of capture molecules immobilized on polystyrene polyaldehyde compared to non treated polystyrene

### 1. Capture probe immobilization

In this example, C4L DNA sequences are used as capture nucleotide sequence. They are produced by PCR as in example 2 but are not biotinylated. They are fixed on the array at a concentration of 300 nM as described in example 2.

### 2. Hybridization and colorimetric detection

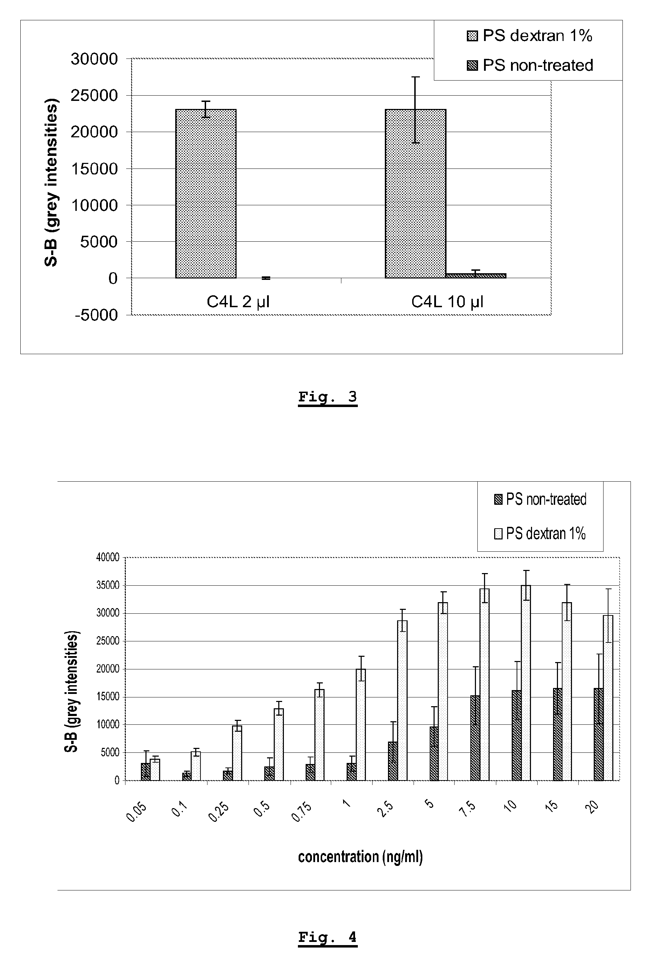

A volume of 2 or 10 µl of Biotin Hyb Control C4L (Eppendorf) is mixed with 10 µl of Hybribuffer A (Eppendorf), 40 µl of Hybribuffer B (Eppendorf) and the volume is adjusted to 100 µl with distilled water. The mix is dispensed into the wells (100 µl/well). Wells are sealed with a plastic coverslip and incubated for 2 h at 65°C. wells are washed 3 times 1 min with washing buffer. Colorimetric detection is performed as described in example 2. Results are presented in figure 3.

### Example 4: Detection of antibodies fixed on polystyrene polyaldehyde

### 1. Capture probe immobilization

Chicken IgY antibodies are used as capture probe. Covalent fixation occurs between free amines of IgY and aldehyde functions of MTP. The aldehyde MTP are obtained as described in example 2 and non treated PS is used as negative control. Decreasing concentrations (ranging from 20 to 0.05 ng/ml) of chicken IgY antibodies are spotted on aldehyde MTP in solutions composed of Mannitol 1%, NP40 0.01%, NaN₃ 0.1%, BBS (borate buffer saline pH 8.5) 0.075M.

### 2. Binding and colorimetric detection

Wash the wells 2 times for 2 min with washing buffer. Dilute primary antibody (Goat anti-chicken IgY biotinylated) 1000 x in blocking buffer. Place the MTP on a thermomixer at a speed of 300 rpm and incubate 1 h at room temperature with primary antibody, 100 µl/well. Wells are washed 3 times for 2 min with washing buffer, then blocked for 10 min in blocking buffer. Colorimetric detection is performed as described in example 2. Results are presented in figure 4.

### Example 5: Unstability of plasma treatment. XPS analysis of cycloolefin immediately after N₂ plasma treatment and after one week of treatment

The plastic sample used is poly-Cycloolefin (Zeonex^{™} 330R) slide of 7.5 x 2.5 cm.
After N₂ plasma treatment, the nitrogen functions and amino groups are identified upon the solid support by X-ray photoelectron spectroscopy (XPS) analysis. The chemical analysis is carried out on C1S and N1S, by fitting the experimental envelope with mixed Gaussian/Lorentzian curves.

### 1. Nitrogen plasma treatment

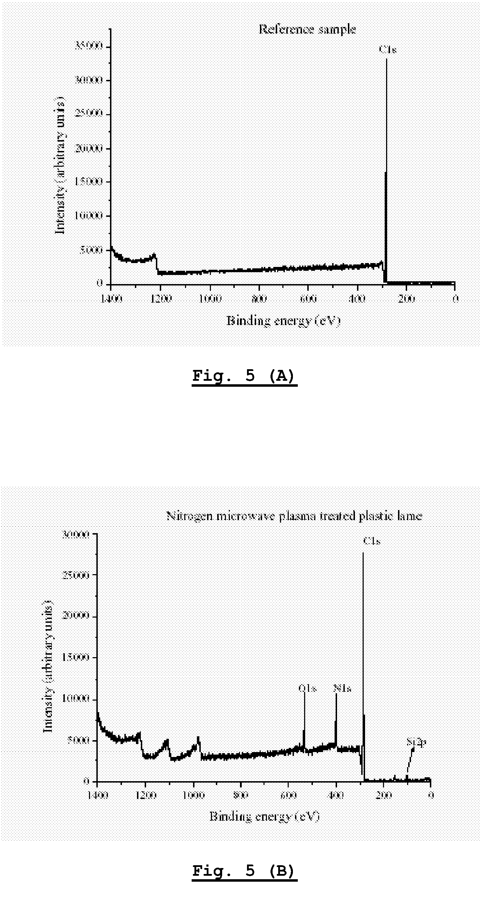

The treatment of the Zeonex^{™} is carried out in late Ar/N₂ remote microwave plasma under a pressure of 3 Torr. This type of source is chosen due to its high degree of dissociation and high concentration of active species (radicals). The sample is placed in the post-discharge at a distance of 27 cm. The total flowrate is 2000 sccm, and the treatment time is 45 min. The gases are considered to be pure (99.9995%). After preparation of the samples, we took 5 x 5 mm piece and introduced it immediately under vacuum in order to analyse it by XPS. After "fresh" analysis, we let the sample ageing for seven day at the ambient atmosphere and analyzed it again.
The XPS measurement shows that the constitutive polymer is a polyolefin, no other peaks than C1s were detected (figure 5A). The general XPS spectrum after "fresh" analysis of the N₂ plasma treatment shows in addition to carbon, nitrogen , oxygen and some traces of silicon (this is due to the sputtering of glass tubes inherent to the plasma modification chamber) (figure 5B).

### 2. Element analysis

After N₂ plasma treatment, the atomic percentage of the detected elements is shown in the next Table.

### Table 1

Atomic percentages of carbon, oxygen, nitrogen and silicon detected on the nitrogen microwave plasma treated Zeonex^{™} slide immediately after treatment and one week after the treatment.

| *Sample* | C1s (% at) | O1s (% at) | N1s (% at) | Si2p(% at) |
|---|---|---|---|---|
| fresh | 73.89 | 9.46 | **14.51** | 2.14 |
| after one week of treatment | 81.05 | 9.14 | **8.12** | 1.01 |

### 3. Chemical analysis

The N1s spectrum allows more easily to make a clear distinction about the amine content of the surface. Table 2 summarizes the characteristics of the nitrogen signal of the nitrogen microwave plasma treated Zeonex^{™} slide immediately after treatment and one week after the treatment.

### Table 2

Binding energy of the components constituting the nitrogen signal, chemical attribution, proportion of N1s signal and corresponding atomic percentages of the nitrogen microwave plasma treated Zeonex^{™} slide.

| *Sample* | Nls | % atomic |
|---|---|---|
| fresh | 399.4 eV | |
| | -(NH)ₓ-(CH)_{y}-, N=(CH)ₓ- | 7.04 |
| | 400.6 eV -CN | 4.93 |
| | 401.7 eV -NH-(C=O)- | 2.54 |
| after one week 399.4 eV | | |
| of treatment | -(NH)ₓ-(CH)_{y}-, N=(CH)ₓ- | 1.8 |
| | 400.6 eV -CN | 2.8 |
| | 401.7 eV -NH-(C=O)- | 3.5 |

In the fresh sample, 14.5 % atomic nitrogen was detected by the method in which 7.04 are under the form if amine and/or imine functions. After ageing of this fresh sample during 7 days only 1.8% of N1S component was still associated to amine function.
This XPS analysis shows that a surface treated by N₂ plasma treatment is unstable with time. A significant decrease of the nitrogen concentration is observed only one week after the treatment. This behaviour is due to the loss of nitrogen functions and/or to a reorganisation of the mobile polymer chains.

### Example 6: Stability with time of cycloolefin polyaldehyde slides stored at 4°C or at 40°C.

The hybridization capacity of capture molecules immobilized on COC Zeonex^{™} 330R polyaldehyde is compared with a surface of COC Zeonex^{™} 330R treated by NH₃ plasma without dextran treatment (negative control) and with a surface of glass carrying aldehydes (diaglass, positive control).

### 1. Preparation of COC polyaldehyde

Preparation of COC polyaldehyde slide is performed as described in example 1 for polystyrene microtiter plate.

### 2. Capture probe immobilization

After 1 day of storage, 1, 2, 3 week and 1 , 2, 3 months, the Zeonex^{™} slides functionalized with polyaldehyde are spotted with capture probes and the capture probes is tested for their hybridization capability.

In this example, AF-583 (nitric oxide synthase 2A) DNA sequences are used as capture nucleotide sequence. They are produced by PCR as in example 2 but are not biotinylated. They are fixed on the array at a concentration of 300 nM as described in example 2.

### 3. Hybridization and colorimetric detection

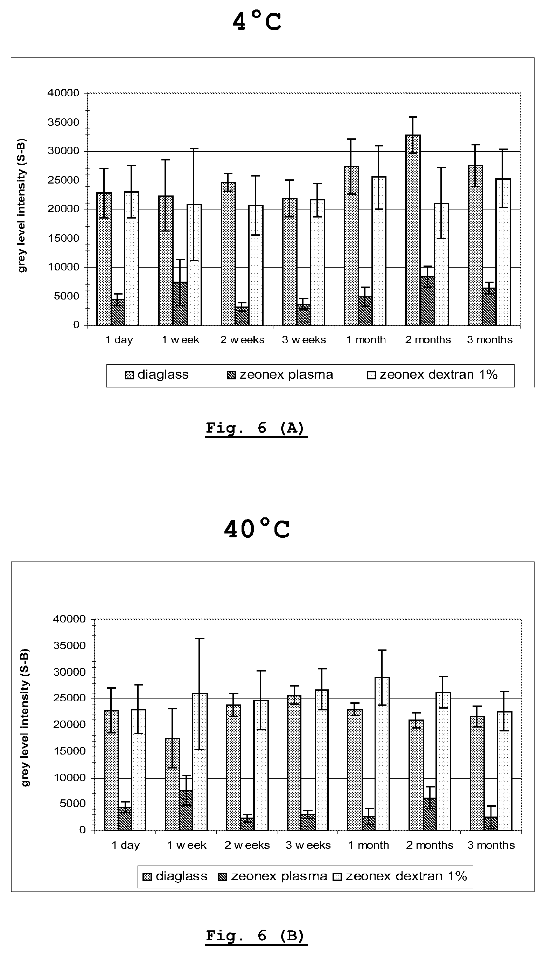

A volume of 2 µl of biotinylated probe AF-583 is mixed with 2 µl of biotin hybridization control C4L, 6.5 µl of Hybribuffer A (Eppendorf), 26 µl of Hybribuffer B (Eppendorf), 3.25 µl of Denhardt and the volume is adjusted to 65 µl with distilled water. The array spotted on Zeonex^{™} 330R polyaldehyde is surrounded by an hybridization frame (Eppendorf). The mix is dispensed into the hybridization frame. The slide is incubated for 2 h at 65°C. The slide is washed 3 times 1 min with washing buffer. Colorimetric detection is performed as described in example 2 except that the slide is placed in a box containing the Anti biotin-gold conjugate 500x diluted in blocking buffer before being placed in the thermomixer. Results are presented in figure 6. The Zeonex^{™} slide polyaldehyde is stable for at least 3 months at 40°C. The slide used as negative control (Zeonex^{™} plasma treated) gives a much lower binding capacity. The slide used as positive control (diaglass) gives a binding capacity comparable to the Zeonex^{™} polyaldehyde.
Unexpectedly, the slide after ammonia plasma treatment alone shows a very high background in colorimetry. The signal value for the background after 1 day storage at 4°C (experiment of figure 6) corresponds to 19944 of grey level intensity. This means that the plasma treatment introduces other functional groups than the desired ones and these other functions are responsible for non-specific DNA binding. After dextran treatment, the background is reduced to a value of 2841 of grey level intensity. Thus, the dextran treatment has a double function; it stabilizes the amino groups introduced by plasma and also avoids non specific binding of DNA with the other functions introduced by plasma.

## Claims

1. A method for obtaining microarrays on a polymeric solid support surface comprising the steps :
- activating a surface of the polymeric solid support by a plasma treatment in order to allow the formation of amino groups comprising primary amino groups;
- providing a macromolecule bearing multiple aldehyde groups,
- covalently binding the macromolecule on the surface of the solid support, wherein said macromolecule is immobilized by at least one shift base or imine group,
- covalently fixing upon the free aldehyde groups of the immobilized macromolecule, capture molecules carrying one or more amino groups by a deposition of a solution containing the capture molecules on the support, wherein the covalent fixation results in an array comprising a density of at least 4 discrete regions per cm² of solid support surface, each of said discrete surface regions being fixed with a species of capture molecules.

2. The method according to the claim 1, wherein the amount of capture molecules fixed in one discrete region varies by less than 25%, and preferably less than 10% as compared to the amount fixed in a replica discrete region.

3. The method according to claim 1 or 2, wherein the capture molecules are designed for a detection, an identification, a quantification and/or a recovery of complementary target biological or chemical molecules of interest.

4. The method according to claims 1 to 3, wherein the surface of the discrete regions fixed by the capture molecule is less than 2 mm² and even less than 0.1 mm² and even less than 0.01 mm².

5. The method according to any of the preceding claims, wherein the density of the discrete region is higher than 20 and even higher than 100 and even higher than 1000 per cm².

6. The method according to any of the preceding claims, wherein the fixation of the capture molecule after 1 and better 3 months does not decrease more than 25 %, when the solid support is kept at 4°C.

7. The method according to any of the preceding claims, wherein the fixation of the capture molecule after 1 and better 3 months does not decrease more than 25%, when the solid support is kept at 40°C.

8. The method according to any of the preceding claims, wherein the density of the capture molecules fixed on the solid support is at least 20 fmoles and better 200 fmoles of capture probes/per cm².

9. The method according to any of the preceding claims, wherein the solution containing the capture molecules deposited on the support does not contain any chemical activation molecule.

10. The method according to any of the preceding claims, wherein the fixation of the capture molecules on the support is performed within 10, better 5 and even better 2 minutes.

11. The method according to claim 1, wherein an obtained fluorescence detection signal of the solid support is decreased by a factor of at least 4, after a binding of the macromolecule compared to a plasma activated polymer.

12. The method according to any of the preceding claims, wherein the macromolecule has a molecular weight comprised between 10000 and 1000000, preferably between 50000 and 100000 daltons.

13. The method according to any of the preceding claims, wherein the macromolecule is an oxidized polysaccharide.

14. The method according to claim 12, wherein the macromolecule is dextran or agarose.

15. The method according to any of the preceding claims, wherein the solid support surface comproses or is made of polymer selected from the group consisting of: polystyrene (PS), polycarbonate (PC), Polymethyl methacrylate (PMMA), polyethylene (PE), Cycloolefin copolymer (COC), cyclic olefin polymer (COP) or a mixture thereof

16. The method according to any of the preceding claims, wherein the solid support comprises multiple microarrays surfaces disposed according to a multiple wells microtitre plate format.

17. The method according to claim 16, wherein the multiple wells microtitre plate format is selected from the group consisting of 6-wells, 12 wells, 24-wells, 48-wells or 96-wells formats.

18. The method according to any of the preceding claims, wherein the plasma treatment is nitrogen or ammonia plasma treatment.

19. The method according to claims 1, wherein the capture molecules are biological capture molecules.

20. The method according to claim 19, wherein the biological capture molecules are selected from the group consisting of: antibodies (or hypervariable portion(s) thereof)/antigens (or haptens), receptors/ligands or nucleic acids (single or double strand DNA or RNA fragments).

21. The method according to any of the preceding claims, wherein the capture molecules are chemical molecules able to bind specifically target chemical molecules obtained by combinatorial chemistry.

22. A microarray, comprising a polymeric solid support having bound on its surface a macromolecule bearing aldehyde functional groups, said macromolecule being bound to the surface of the polymeric support by at least one shift base or imine group and upon which capture molecules carrying one or more amino groups are covalently fixed on a macromolecule, said covalent fixation resulting in an array comprising a density of at least 4 discrete regions/cm² of solid support surface, each of said discrete surface regions being fixed with a species of capture molecules.

23. The microarray of claim 22, wherein the density of fixed capture molecule(s) on the solid support surface is higher than 20 fmoles of capture molecules per cm².

24. The microarray of claim 22 or 23, wherein the density of fixed capture molecule(s) on the solid support surface is higher than 200 fmoles of capture molecules per cm².
